# EUROPÄISCHE PATENTSCHRIFT

(11) **EP 0 342 380 B1**
(45) Veröffentlichungstag und Bekanntmachung des Hinweises auf die Patenterteilung: **03.02.1993**
(21) Anmeldenummer: 89107196.1
(22) Anmeldetag: 21.04.1989
(51) Int. Cl.: A61K 7/18

(54) **Zahncremes und Verfahren zur Stabilisierung**
Toothpastes and process to stabilize them
Pâtes dentifrices et procédé pour leur stabilisation

(30) Priorität: 19.05.1988 DE 3817014
(43) Veröffentlichungstag der Anmeldung: 23.11.1989
(73) Patentinhaber: Württembergische Parfümerie-Fabrik GmbH, 73054 Eislingen (DE)
(72) Erfinder: Scheller, Hans-Ulrich, Dr., D-7332 Eislingen (DE); Scheller, Karl Alexander, Dipl.-Volksw., D-7332 Eislingen (DE)
(74) Vertreter: Werner, Hans-Karsten, Dr.Dipl.-Chem.

(56) Entgegenhaltungen:
- US-A- 4 327 079
- US-A- 4 342 741
- US-A- 4 684 518

## Beschreibung

Zahncremes enthalten als Putzkörper hauptsächlich Calciumcarbonat oder Dicalciumphosphate. Weiterhin werden auch amorphe Kieselsäuren verwendet sowie Aluminiumoxyhydrat und "Madrell'sches Salz". Insbesondere zur Behandlung überempfindlicher Zähne wurden von der Anmelderin Zahncremes entwickelt mit einem hohen Gehalt an Hydroxylapatit. Sofern derartigen Zahncremes zusätzlich lösliches Fluorid zugesetzt wird, beobachtet man eine starke Fluorzehrung, d.h. der Gehalt an löslichem Fluorid sinkt von dem ursprünglich zugegebenen Gehalt innerhalb von Wochen auf unter die Hälfte und im Laufe von Monaten auf unter 10% der ursprünglich zugesetzten Menge.

Eingehende Untersuchungen der Anmelderin haben zunächst den Verdacht bestätigt, daß diese Fluorzehrung darauf beruht, daß Hydroxylapatit sich mit löslichem Fluorid in Fluorapatit umwandelt.

Aus Fey, Otte, "Wörterbuch der Kosmetik" (1985), 2. Auflage, Seite 294, linke Spalte, Zeilen 16 bis 22 geht hervor, daß zur Verhinderung der hydrolytischen Spaltung von Dicalciumphosphat Magnesiumphosphat und/oder Salze kondensierter Phosphorsäuren (Na₄P₂O₇) zugesetzt werden. Dies erfolgt insbesondere bei fluorhaltigen Zahnpasten, um die Bildung von schwerlöslichem Calciumfluorid zu verhindern. Die gattungsgemäßen Zahncremes enthalten jedoch Hydroxylapatit, welches sich durch lösliche Fluoride in Fluorapatit umwandelt. Diese Fluorzehrung ist wesentlich stärker und intensiver als bei Zahncremes, die nur Dicalciumphosphat als Putz- und Poliermittel enthalten und aus denen schwerlösliches Calciumfluorid entsteht. Die weitere Untersuchung der Stabilität von Zahncremes durch Zusatz von Natronlauge und Einstellung des pH-Wertes auf 8,8 hat gezeigt, daß durch diese Maßnahmen die Bildung von Fluorapatit praktisch nicht gebremst werden kann, so daß das Problem der Fluorzehrung hierdurch nicht gelöst werden kann.

Die besondere Problematik von Zahncremes ist, daß es sich weder um verdünnte Lösungen noch um trockene Pulver handelt, sondern um verschiedene Phasen, die bezüglich aller löslichen - wenn auch schwerlöslichen - Komponenten gesättigt oder übersättigt sind. In derartigen Systemen sind Vorhersagen über das Verhalten einzelner Komponenten nicht möglich.

Die Erfindung hat sich somit die Aufgabe gestellt, den Gehalt an löslichem Fluorid in Zahncremes mit einem Gehalt an Hydroxylapatit zu stabilisieren, dabei jedoch nicht die Wirksamkeit und Verträglichkeit der Zahncreme zu beeinträchtigen.

Es wurde jetzt gefunden, daß diese Aufgabe überraschend einfach dadurch gelöst werden kann, daß man der Zahncreme 0,5 bis 5 Gew.-% neutrales Natriumpyrophosphat zusetzt.

Die Wirkungsweise des neutralen Natriumpyrophosphats ist noch nicht bekannt und beruht mit Sicherheit nicht auf der Veränderung des pH-Wertes, da bei einer entsprechenden Veränderung des pH-Wertes, beispielsweise durch Natronlauge, keine entsprechende Stabilisierung beobachtet wird.

Der Gehalt an löslichem Fluorid in den erfindungsgemäßen Zahncremes wird durch den Zusatz von neutralem Natriumpyrophosphat dahingehend stabilisiert, daß nach 3 Wochen noch mindestens 80%, nach 3 Monaten noch mindestens 70% und nach 17 Monaten noch ca. 50% des ursprünglich zugesetzten freien Fluorids vorhanden sind.

Gegenstand der vorliegenden Erfindung sind somit Zahncremes enthaltend Hydroxylapatit und lösliches Fluorid, dadurch gekennzeichnet, daß sie zur Stabilisierung des löslichen Fluoridgehaltes zusätzlich 0,5 bis 5 Gew.-% neutrales Natriumpyrophosphat enthalten.

Ein weiterer Gegenstand der vorliegenden Erfindung ist das Verfahren zur Stabilisierung des löslichen Fluoridgehaltes in Zahncremes mit einem Gehalt an Hydroxylapatit, dadurch gekennzeichnet, daß 0,5 bis 5 Gew.-% neutrales Natriumpyrophosphat zugesetzt wird.

Schließlich ist Gegenstand der vorliegenden Erfindung die Verwendung von 0,5 bis 5 Gew.-% neutralem Natriumpyrophosphat zur Stabilisierung des löslichen Fluoridgehaltes in Zahncremes mit einem Gehalt an Hydroxylapatit.

Bei einem Gehalt von weniger als 0,5 Gew.-% neutralem Natriumpyrophosphat findet noch keine ausreichende Stabilisierung des Gehaltes an löslichem Fluorid statt. Bei einem Gehalt von mehr als 5 Gew.-% neutralem Natriumpyrophosphat wird eine unerwünschte Erhöhung des pH-Wertes über pH 10 beobachtet. Vorzugsweise beträgt somit der Gehalt 1 bis 3 Gew.-% neutrales Natriumpyrophosphat.

Das lösliche, d.h. frei verfügbare Fluorid wird bestimmt, indem Zahnpasta mehrfach mit Wasser dispergiert und zentrifugiert wird. Die vereinigten Zentrifugate werden mit Salzsäure versetzt und in der so erhaltenen Losung der Fluoridgehalt mit einer fluoridsensitiven Elektrode gemessen. Untersuchungen mit einer handelsüblichen Zahncreme der Anmelderin mit einem Gehalt an Hyroxylapatit ergaben, daß bei Zusatz von Fluoridverbindungen der Gehalt an löslichem Fluorid nach 2 bis 3 Wochen auf 40 bis 50% der ursprünglich zugesetzten Menge absinkt und nach 20 Monaten weniger als 10% der ursprünglich zugesetzten Menge beträgt. Hierbei wurden annähernd gleiche Ergebnisse erzielt, unabhängig davon, ob das Fluorid in Form von Natriumfluorid oder Natriummonofluorphosphat zugegeben wird.

Durch Zusatz von Natronlauge und Einstellung des pH-Wertes auf 8,8 wurde zwar eine gewisse Verlangsamung der Fluorzehrung beobachtet. Diese war jedoch völlig ungenügend, um das Problem der Fluorzehrung zu lösen, denn nach 3 Monaten war der Gehalt an freiem Fluorid auf weniger als 50% abgesunken, während die gleiche Rezeptur ohne Zusatz von Natronlauge nach 3 Monaten nur noch ein Drittel des freien Fluoridgehaltes aufwies.

Erst bei Zusatz von neutralem Natriumpyrophosphat ist es gelungen, die Fluorzehrung so zu verlangsamen und zu unterdrücken, daß Zahncremes entstehen, die eine ausreichende Lagerstabilität aufweisen. Bei einem Zusatz von 2,5 Gew.-% neutralem Natriumpyrophosphat zu einer handelsüblichen Zahncreme mit einem Gehalt von 17 Gew.-% Hydroxylapatit zeigte sich, daß bei Zusatz von 1,2 Gew.-% Natriummonofluorphosphat oder 0,33 Gew.-% Natriumfluorid der Gehalt an freiem Fluorid nach 5 Monaten noch mehr als 70% und nach 18 Monaten noch ca. 50% der ursprünglich zugesetzten Menge an löslichem Fluorid beträgt.

Reihenversuche mit steigenden Mengen von neutralem Natriumpyrophosphat haben ergeben, daß bei Zusatz von 0,5 Gew.-% schon eine deutliche Stabilisierung eintritt. Eine für die Praxis akzeptable Stabilisierung wird bei Zusatz von 1 Gew.-% beobachtet. Bei Zusatz von 3,5 bis 5 Gew.-% wird eine weitere Steigerung der Stabilisierung beobachtet, jedoch steigen die pH-Werte der Rezepturen deutlich an. Bei Zusatz von mehr als 5 Gew.-% wird der als zahnmedizinisch noch akzeptabel angesehene pH-Wert von 10 überschritten.

## Patentansprüche

1. Zahncremes enthaltend Hydroxylapatit und lösliches Fluorid, dadurch gekennzeichnet, daß sie zur Stabilisierung des löslichen Fluoridgehaltes zusätzlich 0,5 bis 5 Gew.-% neutrales Natriumpyrophosphat enthalten.

2. Verfahren zur Stabilisierung des löslichen Fluoridgehaltes in Zahncremes mit einem Gehalt an Hydroxylapatit, dadurch gekennzeichnet, daß 0,5 bis 5 Gew.-% neutrales Natriumpyrophosphat zugesetzt wird.

3. Verwendung von 0,5 bis 5 Gew.-% neutralem Natriumpyrophosphat zur Stabilisierung des löslichen Fluoridgehaltes in Zahncremes mit einem Gehalt an Hydroxylapatit.

## Claims

1. Toothpastes containing hydroxylapatite and soluble fluoride characterized in that they additionally contain neutral sodiumpyrophosphate of 0.5 to 5% by weight for stabilization of the soluble fluoride contents.

2. A process for stabilization of the soluble fluoride contents in toothpastes having an amount of hydroxylapatite, characterized in that neutral sodiumpyrophosphate of 0.5 to 5% by weight is added.

3. The use of 0.5 to 5% by weight of neutral sodiumpyrophosphat to stabilize the soluble fluoride contents in toothpastes having an amount of hydroxylapatite.

## Revendications

1. Des pâtes dentifrices contenant d'hydroxylapatite et de fluorure soluble caractérisées en ce qu'elles contiennent en plus de 0,5 à 5% en poids de pyrophosphate de sodium neutre pour la stabilisation de la teneur en fluorure soluble.

2. Procédé pour la stabilisation de la teneur en fluorure soluble dans les pâtes dentifrices contenant une quantitée de l'hydroxylapatite, caractérisé en ce que il est ajouté en poids 0,5 à 5% du pyrophosphate de sodium neutre.

3. Utilisation de 0,5 à 5% en poids du pyrophosphate de sodium neutre pour la stabilisation de la teneur en fluorure soluble dans les pâtes dentifrices contenant une quantitée de l'hydroxylapatite.
